# EUROPEAN PATENT APPLICATION

(11) **EP 3 613 333 A1**
(43) Date of publication of application: **26.02.2020**
(21) Application number: 18792336.2
(22) Date of filing: 20.04.2018
(51) Int. Cl.: A61B 1/012, A61B 1/05, A61B 1/00

(54) **ENDOSCOPE WITH AN ENLARGEABLE TAIL END**

(30) Priority: 27.04.2017 CN 201710286465
(71) Applicant: Sihong Zhengxin Medical Technology Co., Ltd., Suqian, Jiangsu 223900 (CN)
(72) Inventor: ZHENG, Yang, Nanjing Jiangsu 210004 (CN); ZHENG, Xing, Nanjing Jiangsu 210004 (CN)
(74) Representative: Rapisardi, Mariacristina
(86) International application number: PCT/CN2018/083940
(87) International publication number: WO 2018/196697

(57) **Abstract**

Disclosed is an endoscope with an enlargeable distal end, including an inserting portion and an endoscope main body. The inserting portion includes a housing, a working cavity, a camera, and an wire band. The working cavity, the camera and the wire band are all disposed in the housing. One end of the wire band is connected to the camera, and the other end the wire band passes through the housing to be connected to the endoscope main body. The camera is arranged at a distal end in the housing, where there is provided a notch. The working cavity or the camera can extend out from or retract back into the notch. When the working cavity or the camera extends out from the notch, the working cavity and the camera are situated side by side. The endoscope with the enlargeable distal end can achieve the enlargement and retraction of the distal end of the endoscope. The distal end of the endoscope is enlarged after passing through a narrow human body tract so that the working cavity or the camera extends out and the working cavity or the camera are posed side by side, thereby significantly increasing an inner diameter of the working cavity, allowing more instruments to pass through to take out larger foreign matters, and providing more ideal 3D images.

## Description

### TECHNICAL FIELD

The present disclosure relates to an endoscope with an enlargeable distal end, which belongs to the field of medical devices.

### BACKGROUND

An endoscopy is a common medical device, usually including an inserting portion that is inserted deep into a body cavity, and an endoscope main body. A camera module is arranged in the inserting portion for image capturing purposes. Image signals output from the camera module are transmitted to the endoscope main body outside the human body for further processing, and then transmitted to a back-end control and display circuitry to facilitate a doctor with detection and related operation. Nowadays, endoscopes come in a variety of types, such as thoracoscopy, laparoscopy, hysteroscopy, rhinoscope, otoscopy, etc.

A common problem of all existing endoscopes lies in a small inner diameter of the working cavity hence a narrow application range. This is related to the traditional structure of the endoscope. In particular, human body cavities are generally thin and have the sphincter that functions as a safeguard, so that if the endoscope wants to smoothly pass through a cavity, the inserting portion of the endoscope must also be very fine and have a certain hardness. The insertion porting is also the acting end of the endoscope, and needs to be provided with a lighting LED or light guide bundle, a camera module, a flush or inflation passage, and a device passage tube. These devices need to be disposed side by side on the fine top, so the device passage must be very small, making it difficult to obtain ideal 3D effects.

Taking the small flexible ureteroscopy that falls in the endoscope category as an example. The common Olympus electronic ureteroscopy is F8, in which a an acting end has a diameter of 2.55 mm, a stainless steel shell has a thickness of 0.15 mm, two sides of which total to 0.3mm, and a camera has a diameter of about 1 mm. Except for the space occupied by a light guide bundle and a bending regulating steel wire rope, the maximum allowable inner diameter for a device passage is only about 1.2 mm, which only allows a device as thin as a toothpick to pass through. Therefore, such type of endoscope is usually only used for observation, biopsy, and laser lithotripsy. For a surgery requiring the use of two devices simultaneously, a laparoscope and open surgery would be needed.

The high price is also a big problem of the endoscope. The endoscope has high requirements for sealing and waterproofness, because the body fluids entering the endoscope will corrode snake-type bones and internal pipelines, and proteins will adhere to and block moving parts, so that the endoscope may fail to work properly when used again. The bending portion of a flexible endoscope consists of multiple snake-like joints connected to each other, and their connecting structures allow these joints to pivot relative to one another. The connecting structures are arranged on surfaces of these snake-like joints. These snake-like joints and connecting structures are complex to manufacture, and the connection and assembly of these snake-like joints are also very complicated, leading to a high cost.

A utility model No. 200780040247.4 entitled "Construction for a Bending Section of an Endoscope" discloses a structure for a bending section of an endoscope. In this utility model, first steps are provided on both sides of two projecting plates disposed in the front side of the front end section of a node ring and are configured for limiting a swing angle. Furthermore, second steps are provided on both sides of two projecting plates disposed in the rear side of the rear end section of the node ring and are configured for limiting the swing angle. When the bending section is bent to the degree that two node rings of the bending tube one in front of the other are rotated to a maximum rotation position, then the second steps of the front node ring and the first steps of the rear node ring would abut against each other thereby limiting further movement of the node rings. Therefore, the swing angle A formed by the front and rear node rings is limited to a predetermined limitation angle at which an outer sleeve tube is prevented from being caught between the node rings when bending the bending section. Thus, the outer sleeve tube is prevented from being caught between the node rings when bending the bending section. However, this utility model is complex in structure, difficult to form, and has a high cost.

### SUMMARY

In order to solve the problems of the endoscopes in the related art, such as a small inner diameter of the working cavity, a narrow application range, a high cost, difficulty to maintain, and vulnerability to wear, the present disclosure provides an endoscope with an enlargeable distal end.

To solve the above technical problems, the present disclosure adopts the following solutions.

There is provided an endoscope with an enlargeable distal end which includes an inserting portion and an endoscope main body. The inserting portion includes a housing, a working cavity, a camera, and an wire band. The working cavity, the camera and the wire band are all disposed in the housing. One end of the wire band is connected to the camera, and the other end of the wire band passes through the housing to be connected to the endoscope main body. The camera is disposed on a distal end in the housing. The distal end of the housing is provided with a notch. The working cavity or the camera can extend out from or retract back into the notch. When the working cavity or the camera extends out from the notch, the working cavity and the camera are situated side by side.

Typically, an axis of the working cavity and an axis of the camera i form an angle that lies in the range of 0 to 30°. Exemplarily, the working cavity and the camera may run parallel to each other.

Typically, the distal end of the housing is provided with at least two notches, and in a front or rear side of the camera there is arranged at least one surgical component that is extendable outward from or retractable back into the notch, such as a camera, a B-mode ultrasound probe, a motor, or an ablation device, etc. Exemplarily, in the same endoscope main body there are arranged at least two cameras that are disposed one in front of the other and that are extendable outward from or retractable back into the notches. Each camera is connected to a corresponding wire band. Signals obtained by each camera are processed by a computer to form a 3D image.

Typically, the distal end of the housing of the endoscope is made of a hard material, and may further be made of a metal or ceramic. It is used for supporting a space in a soft human cavity, to provide a visual field for the camera and prevent the mucous from sticking onto the lens, so that the camera can better perform observation.

In the endoscope of the present application, the outer diameter of the inserting portion is significantly decreased and the inner diameter of the working cavity is significantly increased.

In the present application, the distal end is an acting end (i.e., a working end) of the endoscope. The above working cavity may be an added independent tube, and may also be a space in the housing other than the wire band. When the working cavity can extend out from or retract back into the notch, the working cavity is an added independent tube. In this case, in order to improve stability of use, the camera is typically bonded or otherwise fixed at the distal end of the housing. When the camera can extend out from or retract back into the notch, the working cavity may be an added independent tube, and may also be a space in the housing other than the wire band, and the working cavity is typically the space in the housing other than the wire band. In order to make it easy for the camera or the working cavity to extend out from or retract back into the notch, a maximum width of the notch measured along a direction perpendicular to a longitudinal axis of the endoscope main body is larger than an outer diameter of the working cavity or the camera.

The end of the inserting portion provided with the camera is an acting end and the other end is an operating end. An operating portion on the operation end can be operated and held. The outer diameter of the section of the housing that extends into the human body is smaller than the outer diameter of the section of the housing that remains outside the human body, and the two sections are connected to each other by a smooth transition. The acting end of the endoscope is bendable, and so can pass curve paths in a patient's body or support the selective movement of the camera mechanism; that is, the acting end of the housing may be made of a soft, bendable material.

The operating portion may come in any shape and size that facilitate people to hold this device, such as an operating portion without a vertical handle, which is suitable for being held vertically, or an operating portion with a vertical handle, which is suitable for being held horizontally, etc. In addition, the operating portion may be provided with various controls that an operator can operate to control the endoscope and its functions.

When the working cavity is a space in the housing other than the wire band, the operating portion is similar to an operating portion of the existing endoscope on which a fluid inlet and outlet valve and a device inlet and outlet valve are disposed. When the working cavity is an added independent tube, a fluid inlet and outlet valve and a device inlet and outlet valve are disposed in the working cavity. The operating portion is provided with a guide groove that is used for the fluid inlet and outlet valve and the device inlet and outlet valve to extend out from the housing of the operating portion and allow the working cavity move back and forth along the guide groove. The operating portion is provided with a locking bottom that can lock up a relative position of the working cavity and the guide-track groove.

In order to save space and expand the inner diameter of the working cavity, the wire band may have a flattened shape and includes wires arranged in in parallel and a wrapper that wraps all the wires together; or the wire band is an integrated structure formed by wires that are arranged in parallel and bonded to each other. In order to save space, the wire band is typically attached to an inner wall of the working cavity or integrated into the inner wall of the working cavity.

The wrapper is typically made of a water-proof material. The camera is generally cylindrical, and pixels of a digital imaging chip is proportional to an imaging area, so the diameter of the camera is difficult to compress and a part of space at the front end of the endoscope is occupied. However, since connected wires in the rear do not need the same amount of space, flat, long carrying pole-shaped wire bands can leave more space to facilitate the movement of devices and device passage tubes.

The lens diameter of the camera may be smaller than that of the digital imaging chip. The diameter of the cylindrical camera is determined by the digital imaging chip. The cylindrical camera sold in the current market is generally provided with multiple miniature LED lighting devices around the lens and between the cylinder walls. The power supply and control of the lighting device are integrated on the digital imaging chip. The present application also uses such shooting and lighting module, which is called the camera in the present application.

Further, an wire band storage groove may be defined in the inner wall of the housing.

As a typical solution of the present application, the endoscope with an enlargeable distal end further includes a wedge block. One end of the wedge block is a plane, and the other end thereof is a bevel. The plane end of the wedge block is connected to the camera, and the bevel end of the wedge block is connected to the working cavity. The working cavity is a tube independent of the housing. The notch in the housing is disposed on one side of the bevel of the wedge block. When the acting end of the endoscope extends into a target position, the working cavity is pushed, slides out along the bevel of the wedge block, extends out from the notch of the housing, and finally is in a position parallel to the camera. In this case, the medical device can extend into the working cavity to perform various operations. After the operation, the working cavity is pulled back to the starting position and pulled out from the body. The movement of the working cavity relative to the housing may be implemented by setting a notch on the operating end of the housing and pushing the working cavity directly with hands, or may be implemented by adding a working member, one end of which is fixed to the working cavity and the other end of which extends out from the housing, and applying force to the other end to push the working cavity.

For ease of operation, an axial angle between the bevel of the wedge block and the wedge block lies in the range of 10 to 45°, and typically lies in the range of 20 to 35°.

In order to enhance the protection and restoring effects, the endoscope with an enlargeable distal end further includes an elastic sleeve. The elastic sleeve is disposed at an end portion of the end of the housing provided with the camera, and wraps the camera and the notch of the housing.

As another typical solution of the present application, the endoscope with an enlargeable distal end further includes a wedge block, a push rod, a circular tube, and a semi tube. The semi tube may have a C-shaped. One end of the wedge block is a plane, and the other end of the wedge block is a bevel. The plane end of the wedge block is connected to the camera, and the bevel end of the wedge block is connected to the working cavity. The notch in the housing is disposed on one side of the bevel of the wedge block. The push rod is disposed in the housing. One end of the circular tube is connected to one end of the push rod, and the other end of the circular tube is rotatably connected to at least one end point of one end of the semi tube. The semi tube is connected to the bevel end of the wedge block. The end of the push rod that is not connected to the circular tube passes through and extends out of the housing.

The cross section of the semi tube is C-shaped, and each end of the semi tube has two end points in the longitudinal direction.

In order to enhance the protection and restoring effects, the endoscope with an enlargeable distal end further includes an elastic sleeve disposed at the end portion of the end of the housing provided with the camera, and wraps the camera, the circular tube and the semi tube. When the acting end of the endoscope extends into the target position, the push rod is pushed, and the circular tube drives the semi tube to move toward the camera and finally makes the semi tube extend out from the notch of the housing, so the parts among the semi tube, the circular tube, the wire band and the housing form the working cavity. In this case, the medical device extends into the working cavity and performs various operations. After the operation, by pulling the push rod or under the compression of the elastic sleeve, the circular tube and the semi tube restore to the starting position and then are pulled out from the body.

As another typical solution of the present application, the endoscope with an enlargeable distal end further includes a push rod, a connecting spring, a circular tube and a semi tube. The cross section of the semi tube is C-shaped. The push rod is disposed in the housing. One end of the circular tube is connected to the push rod, and the other end of the circular tube is connected to at least one end point of one end of the semi tube. One end of the semi tube is attached to a periphery of the camera. One end of the push rod that is not connected to the circular tube passes through and extends out from the housing. One end of the connecting spring is connected to the camera, and the other end of the connecting spring is connected to the semi tube. The connecting spring is in a free state. When the acting end of the endoscope extends into the target position, the push rod is pushed, and the circular tube drives the semi tube to move towards the camera and finally pushes the semi tube away from the camera, so the parts among the semi tube, the circular tube, the wire band and the housing form the working cavity. In this case, the medical device can extend into the working cavity to perform various operations. After the operation, by pulling the pull rod and/or with the resisting force of the spring, the circular tube and the semi tube are restored to the starting position and then are pulled out from the body.

The camera is generally a cylinder, with one end being a working end and the other end being a non-working end.

In the present application, the cross section of the semi tube is C-shaped.

As another typical solution of the present application, the endoscope with an enlargeable distal end further includes a pull rope, a circular tube and an oblique tube. One end of the oblique tube is a circular surface and the other end of the oblique tube is a slanting surface. The pull rope is disposed in the housing. One side of an end of the circular tube is rotatably connected to an inner wall of the housing, and the other side of the end of the circular tube is connected to the pull rope. The other end of the circular tube is connected to the circular surface end of the oblique tube. The slanting surface end of the oblique tube is attached to the inner wall of the housing. The camera, the oblique tube, the circular tube and the pull rope are sequentially connected to each other in the direction from outside to inside. A rotary connecting point between the circular tube and the housing is on the same side as the notch or on an opposite side to the notch. When the acting end of the endoscope extends into the target position, the pull rope is pulled, and the circular tube drives the oblique tube to rotate, so the parts among the semi tube, the oblique tube, the wire band and the housing form the working cavity. In this case, the medical device can extend into the working cavity and perform various operations. After the operation, the pull rope is released, and the circular tube and the oblique tube are restored to the starting position and then are pulled out from the body.

The side and the other side of one end of the circular tube are two points opposite each other at an end portion of the one end of the circular tube.

The pull rope can be made of a soft material, and may also be made of a hard material such as a steel strip. For convenience of operation, the material of the pull rope is typically a hard material.

In the present application, the direction from the camera to the inside of the housing is defined as the direction from the outside to the inside.

In the present application, the oblique tube is equivalent to a circular tube with one end cut at a certain angle of inclination. The angle of inclination refers to an angle that is not perpendicular to the axis of the circular tube. One end of the oblique tube is a circular surface that is perpendicular to the axis, and the other end of the oblique tube is a slanting surface (oval-shaped) that is not perpendicular to the axis direction.

In order to enhance the protection and restoring effects, the endoscope with an enlargeable distal end further includes an elastic sleeve disposed at the end portion of the end of the housing provided with the camera and wraps the camera, the circular tube and the oblique tube.

As another typical solution of the present application, the endoscope with an enlargeable distal end further includes an expansion support rod and an elastic sleeve. One end of the housing provided with the camera is an elastically bendable end. The elastic sleeve is sleeved on a periphery of the elastically bendable end. One end of the expansion support rod is rotatably connected to the camera, and the other end of the expansion support rod is rotatably connected to the housing. A range of rotation of the expansion support rod relative to the axis of the housing lies in the range of 0 to 90°. When the acting end of the endoscope extends into the target position, the wire band is pulled, which makes the camera extend out from the notch of the housing, so the part between the wire band and the housing forms the working cavity. The wire band is continuously pulled, and the limiting function of the expansion support rod makes a periphery of the elastically bendable end a bending structure, so as to achieve the function of a snake-shaped tube. In this case, the medical device can extend into the working cavity and perform various operations. After the operations, with the extrusion of the elastic sleeve, the camera restores to the starting position and then is pulled out of the body.

In the present application, the elastically bendable end can be bent in any direction and at any angle.

In the present application, one end of the wire band is connected to the camera, the other end of the wire band passes through the housing to be connected to the endoscope main body, a part of the wire band between the camera and the endoscope main body extends out from the housing, and for ease of operation, the extended part of the wire band is typically disposed at the operating portion.

In order to improve the convenience and stability of the operation, the endoscope with an enlargeable distal end further includes a wedge block. One end of the wedge block is a plane, and the other end of the wedge block is a bevel. The plane end of the wedge block is connected to the camera, and the bevel end of the wedge block is connected to the working cavity. One end of the expansion support rod is rotatably connected to the camera or the wedge block, and the other end of the expansion support rod is rotatably connected to the housing. In this way, the stability can be improved.

For better protection, the endoscope with an enlargeable distal end further includes an expansion member disposed on the periphery of the camera and the wedge block. One end of the expansion support rob is rotatably connected to both the camera and the expansion member, and the other end of the expansion support rob is rotatably connected to the housing. In this way, better protection can be achieved. The shape of the expansion member is close to the shape of the notch of the housing.

As another typical solution of the present application, the endoscope with an enlargeable distal end further includes an expansion member, an expansion support rod, a pull rope, and an elastic sleeve. One end of the housing provided with the camera is an elastically bendable end. The elastic sleeve is sleeved on a periphery of the elastically bendable end. The pull rope is disposed in the housing. One end of the pull rope is connected to the expansion member, and the other end of the pull rope passes through to be connected to the endoscope main body. The extension member is disposed on a periphery of the camera. One end of the expansion support rod is rotatably connected to the expansion member, and the other end of the expansion support rod is rotatably connected to the housing or the camera. When the acting end of the endoscope extends into the target position, the pull rope is pulled, so the parts among the wire band, the housing, and the expansion member form the working cavity. The pull rope is continuously pulled, and the limiting function of the expansion support rod makes a periphery of the elastically bendable end a bending structure, so as to achieve the function of a snake-shaped tube. In this case, the medical device can extend into the working cavity and perform various operations. After the operations, with the extrusion of the elastic sleeve, the camera restores to the starting position and then is pulled out from the body.

As another typical solution of the present solution, the endoscope with an enlargeable distal end further includes an expansion support rod, an expansion member, a wedge block, and a push rod. The expansion member is disposed on a periphery of the camera. One end of the expansion support rod is rotatably connected to the expansion member, and the other end of the expansion support rod is rotatably connected to the camera. One end of the push rod is connected to the expansion member, and the other end of the push rod passes through and extends out from the housing. One end of the wedge block is a plane, and the other end of the wedge block is a bevel. The plane end of the wedge block is connected to the camera. When the acting end of the endoscope extends into the target position, the push rod is pushed, and the expansion meme and the expansion support rod drive the camera to extend out from the notch of the housing, so the parts between the wire band and the housing form the working cavity. In this case, the medical device can extend into the working cavity and perform various operations. After the operations, the push rod is pulled to make the camera restore to the starting position and then pulled out from the body.

In order to improve the convenience and stability of the operation, the endoscope with an enlargeable distal end further includes a wedge block. One end of the wedge block is a plane, and the other end of the wedge block is a bevel. The plane end of the wedge block is connected to the camera, and the bevel end of the wedge block is connected to the working cavity. One end of the expansion support rod is rotatably connected to the camera or the wedge block, and the other end of the expansion support rod is rotatably connected to the expansion member. In this way, the stability can be improved.

For the better limiting and restoring effects, the endoscope with an enlargeable distal end further includes a connecting spring. One end of the connecting spring is connected to the camera, and the other end of the connecting spring is connected to the housing. A compression spring is in the free state. When the connecting spring extends out from the notch of the housing, the connecting spring is in a stretched state. After the operation, the force on the push rod is removed, and the camera restores to the initial position under the resisting force of the connecting spring.

As another typical solution of the present application, the endoscope with an enlargeable distal end further includes a steel plate and an elastic plate. The steel plate is strip-shaped. The steel plate is slidably connected to the inner wall of the housing. The wire band is disposed between the steel plate and the housing. One end of the elastic plate is connected to an end portion of a distal end of the wire band or is connected to the inner wall of the housing that abuts on the distal end of the wire band. When the acting end of the endoscope extends into the target position, the steel plate is pushed and acts on the elastic plate, so that the camera deviates from the original position and extends out from the notch, and then the parts among the camera, the steel plate and the housing form the working cavity. In this case, the medical device can extend into the working cavity and perform various operations, the steel plate is pulled and leaves the elastic plate, and then the camera restores to the starting position and is pulled out from the body.

The elastic plate is preferably S-shaped.

For better limiting and restoring effects, the endoscope with an enlargeable distal end further includes a connecting spring. One end of the connecting spring is connected to the camera, and the other end of the connecting spring is connected to the housing. The connecting spring is in the free state. The connecting spring performs limiting and restoring functions.

For better protection, limiting and restoring effects, the endoscope with an enlargeable distal end further includes an elastic sleeve disposed at the end portion of one end of the housing provided with the camera, and wraps the camera and the elastic plate.

As another typical solution of the present application, the endoscope with an enlargeable distal end further includes a dual-cavity housing and an elastic plate. The wire band is disposed in one of the two cavities. One end of the elastic plate is S-shaped, and the other end of the elastic plate is strip-shaped. The strip-shaped end of the elastic plate is wrapped in the wire band. An end portion of the S-shaped end of the elastic plate extends out from the chamber where the wire band is located to be connected to the camera. The camera is disposed in the notch of the housing. When the wire band is pulled, the elastic plate deviates from the original position and leaves the notch under the pressure from a cavity partition plate in the housing, and the medical device can pass through the chamber without any wire band and perform various operations.

As another typical solution of the present application, the endoscope with an enlargeable distal end further includes a wedge block, a push rod and an elastic sleeve. One end of the wedge block is a plane, and the other end of the wedge block is a bevel. The plane end of the wedge block is connected to the camera, and the bevel end of the wedge block is slidably connected to the housing. The wedge block and the camera are clamped in the notch of the housing. One end of the push rod is connected to the bevel end of the wedge block and pushes or pulls the wedge block to drive the camera to slide relative to the housing, and the other end of the push rod passes through and extends out from the housing.

The endoscope with an enlargeable distal end further includes an elastic sleeve disposed at the end portion of one end of the housing provided with the camera, and wraps the camera. When the acting end of the endoscope extends into the target position, the push rod is pushed to drive the wedge block to slide, and the camera slides away from the notch, so the parts between the wire band and the housing form the working cavity. In this case, the medical device can extend into the working cavity and perform various operations. After the operation, by pulling the push rod and/or under the compression of the elastic sleeve, the camera restores to the starting position and is pulled out from the body.

As another typical solution of the present application, the endoscope with an enlargeable distal end further includes a push rod, an oblique tube and a semi tube. The cross section of the semi tube is C-shaped. The push rod is disposed in the housing. One end of the oblique tube is a circular surface, and the other end of the oblique tube is a slanting surface. The slanting surface of the oblique tube faces away from the notch of the housing. One side of an end of the circular surface of the oblique tube is rotatably connected to the notch, and the other side of the end of the circular surface of the oblique tube is connected to one end of the push rod. The highest point of one end of the slanting surface of the oblique tube is rotatably connected to one end of the semi tube. One end of the push rod that is not connected to the circular tube passes through and extends out from the housing. The cross section of the semi tube is C-shaped.

The oblique tube is a circular tube with a surface cut obliquely. The angle between the slanting surface and the axis is 30-60°, and is preferably 45°. The slanting surface of the oblique tube can be used as a limit stand when it is in contact with the semi tube.

As another typical solution of the present application, the endoscope with an enlargeable distal end further includes an expansion support rod, an expansion member and a pull rope. The expansion part is disposed on the periphery of the camera. One end of the expansion support rod is rotatably connected to the expansion member, and the other end of the expansion support rod is rotatably connected to the camera. One end of the pull rope is connected to the expansion member, and the other end of the pull rope passes through and extends out from the housing.

When the acting end of the endoscope extends into the target position, the pull rope is pulled, and the expansion meme and the expansion support rod drive the camera to extend out from the notch of the housing, so the parts between the wire band and the housing form the working cavity. In this case, the medical device can extend into the working cavity and perform various operations. The wire band is continuously pulled, and the limiting function of the expansion support rod makes a periphery of the elastically bendable end a bending structure, so as to achieve the function of a snake-shaped tube. In this case, the medical device can extend into the working cavity and perform various operations. After the operation, with the extrusion of the elastic sleeve, the expansion support rod restores to the starting position and then is pulled out the body. One end of the wire band is connected to the camera, and the other end of the wire band passes through the housing and is connected to the endoscope main body. The extended part of the wire band is disposed at the operating portion for ease of operation such as pulling and the like.

As another typical solution of the present application, the endoscope with an enlargeable distal end further includes a wedge block. One end of the wedge block is a plane, and the other end of the wedge block is a bevel. The plane end of the wedge block is connected to the camera, and the bevel end of the wedge block is connected to the working cavity. The working cavity is a tube independent of the housing. The notch on the housing is disposed on the opposite side of the bevel of the wedge block.

When the acting end of the endoscope extends into the target position, the working cavity is pushed forward, the working cavity moves forwards along the bevel of the wedge block, pushes the wedge block and the camera out from the notch of the housing, and it is in a position parallel to the camera. In this case, the medical device can extend into the working cavity and perform various operations, and after the operation, the working cavity is pulled back to the starting position and pulled out of the body. The movement of the working cavity relative to the housing may be implemented by setting a notch on the operating end of the housing and pushing the working cavity directly with hands, or may be implemented by adding a working member, one end of which is fixed to the working cavity and the other end of which extends out from the housing, and applying force to the other end to push the working cavity.

As another typical solution of the present application, the endoscope with an enlargeable distal end further includes an elastic plate and an elastic sleeve. One end of the elastic plate is connected to the inner wall of the housing, and the other end of the elastic plate is connected to the camera and the pull rope. A limit stand is disposed at a joint between the elastic plate and the camera. The pull rope passes through the housing and is connected to a control handle. The elastic sleeve is sleeved at the end portion of the end of the housing provided with the camera and wraps the camera. The elastic sheet is preferably U-shaped.

When the acting end of the endoscope extends into the target position, the pull rope is pulled and acts on the elastic plate to make it bend, so that the camera deviates from the original position and extends out from the notch. The limit stand supports the camera. The oblique angle of the limit stand is 15-75°, and is preferably 45°. The part between the wire band and the housing forms the working cavity. In this case, the medical device can extend into the working cavity and perform various operations. The pull rope is released, and the camera restores to the starting position under the action of the elastic sleeve and is pulled out from the body.

Typically, the housing is made of a metal material or a metal mesh material, which provide better shielding effects, thereby making the wire band smaller and finer and increasing the space of the working cavity.

In the present application, the endoscope may be produced as a disposable device or reusable devices depending on requirements.

The technology not mentioned in the present disclosure is referred to the related art.

The endoscope with an enlargeable distal end in the present application can achieve the enlarging and retracting of the distal end of the endoscope. The distal end of the endoscope is enlarged after passing through a narrow human body tract so that the working cavity or the camera extends out and the working cavity or the camera is in a parallel position. The inner diameter of the working cavity is significantly increased. Therefore, more devices can pass through to take out larger foreign matters, the washing is performed more easily, the maintenance becomes simple, and the consumption is less; the production cost is low because it can be used once and can also be used multiple times; and structure design is reasonable, the endoscope is easy to use, and an ideal 3D effect can be provided.

### BRIEF DESCRIPTION OF DRAWINGS

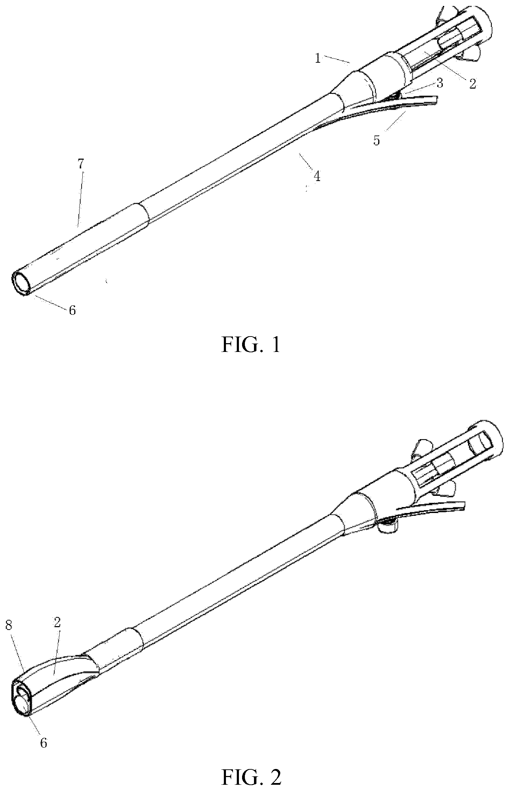

FIG. 1 is a structural diagram of an endoscope with an enlargeable distal end according to Embodiment 3 of the present disclosure.
FIG. 2 is a structural diagram of a working cavity extending out from a notch of a housing and posing parallel to a camera in FIG. 1.

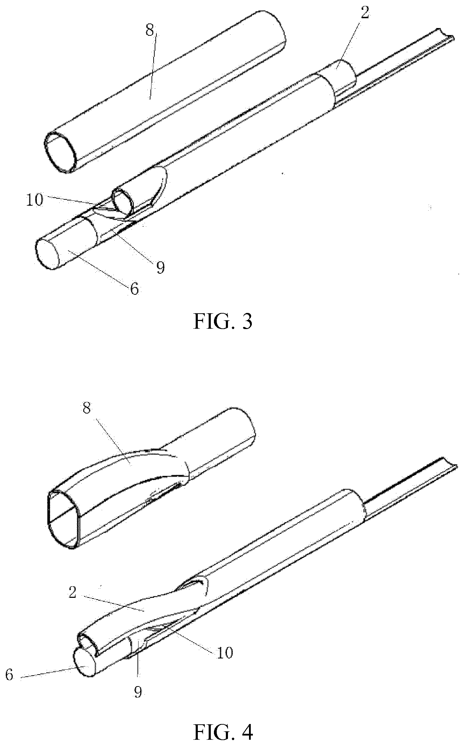

FIG. 3 is an exploded view of a working end of FIG. 1.
FIG. 4 is an exploded view of a working end of FIG. 2.

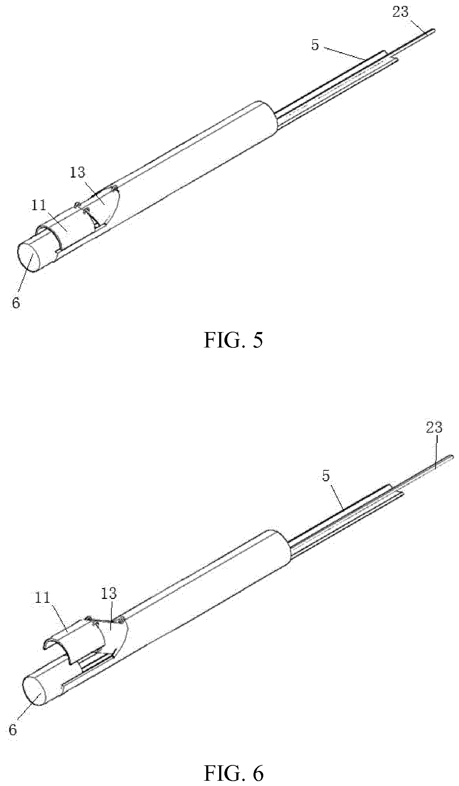

FIG. 5 is a structural diagram of an endoscope with an enlargeable distal end according to Embodiment 4 of the present disclosure.
FIG. 6 is a schematic diagram of a working state of an endoscope with an enlargeable distal end according to Embodiment 4 of the present disclosure.
FIG. 7 is a schematic diagram of a principle of an endoscope with an enlargeable distal end according to Embodiment 5 of the present disclosure.
FIG. 8 is a structural diagram of an endoscope with an enlargeable distal end according to Embodiment 7 of the present disclosure.

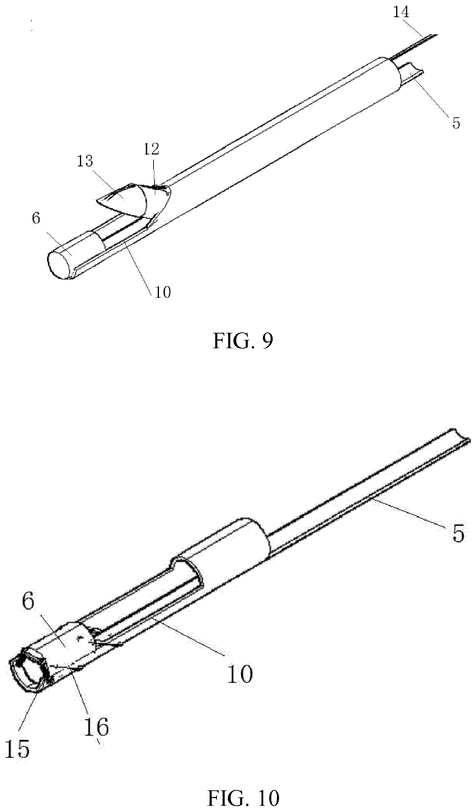

FIG. 9 is a schematic diagram of a working state of an endoscope with an enlargeable distal end according to Embodiment 7 of the present disclosure.
FIG. 10 is a schematic diagram of an endoscope with an enlargeable distal end according to Embodiment 10 of the present disclosure.

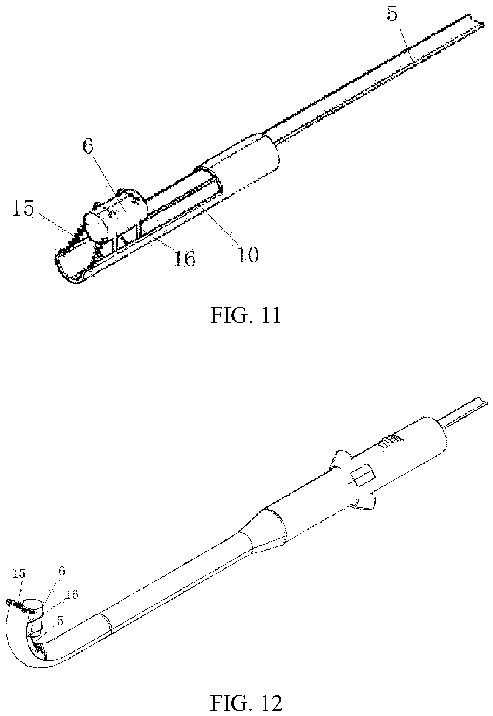

FIG. 11 is a schematic diagram of a pulling process of the endoscope with an enlargeable distal end according to Embodiment 10 of the present disclosure.
FIG. 12 is a schematic diagram of a bending state of an elastically bendable end that continues to be pulled according to Embodiment 10 of the present disclosure.

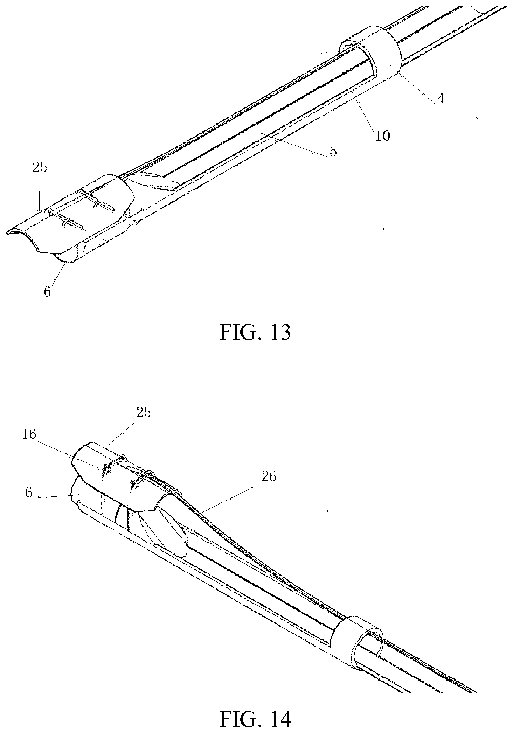

FIG. 13 is a structural diagram of an endoscope with an enlargeable distal end according to Embodiment 13 of the present disclosure.
FIG. 14 is a schematic diagram of a working state of an endoscope with an enlargeable distal end according to Embodiment 13 of the present disclosure.

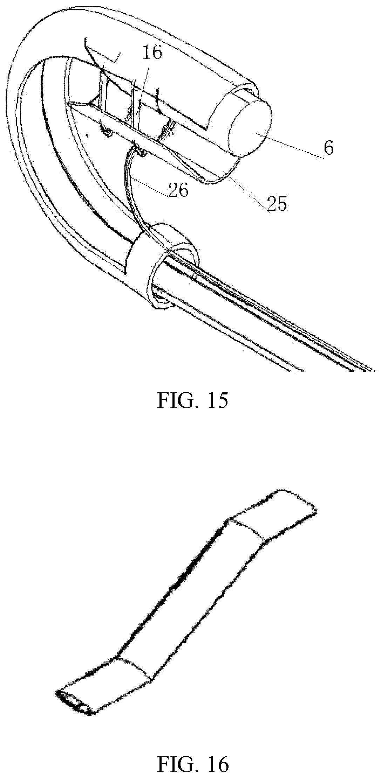

FIG. 15 is a schematic diagram of a bending state of a distal end (a working end) according to Embodiment 13 of the present disclosure.
FIG. 16 is a structural diagram of an elastic plate according to Embodiment 16 of the present disclosure.

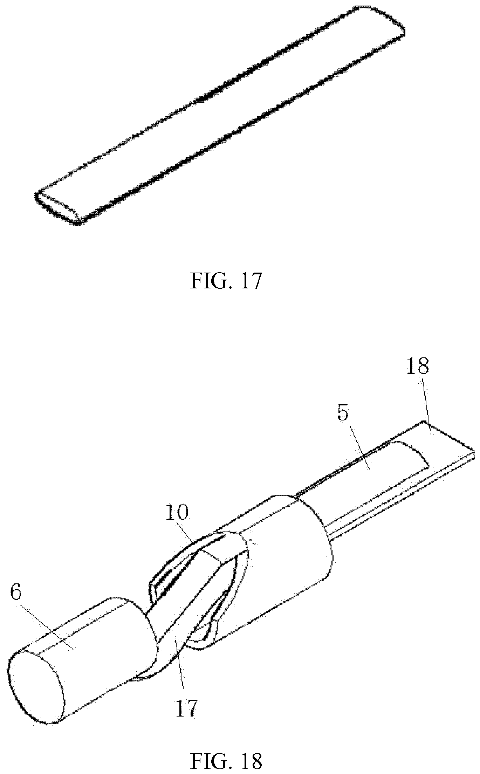

FIG. 17 is a schematic diagram of a stretched state of an elastic plate according to Embodiment 16 of the present disclosure.
FIG. 18 is a structural diagram of an endoscope with an enlargeable distal end according to Embodiment 16 of the present disclosure.

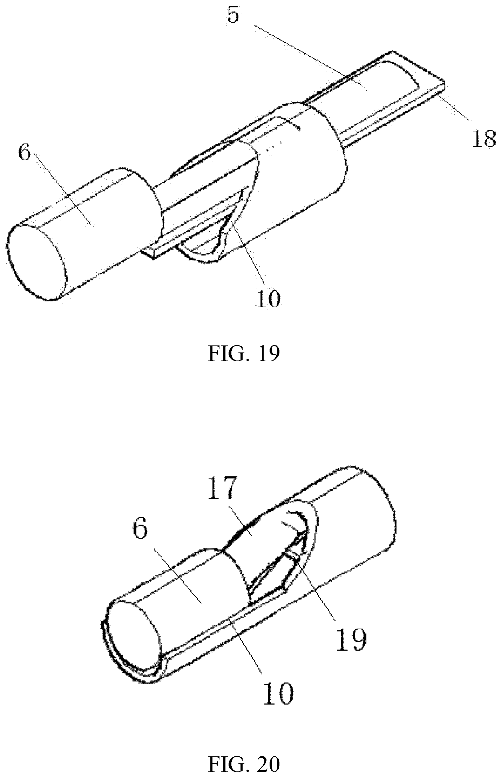

FIG. 19 is a schematic diagram of a working state of an endoscope with an enlargeable distal end according to Embodiment 16 of the present disclosure.
FIG. 20 is a schematic diagram of an endoscope with an enlargeable distal end according to Embodiment 17 of the present disclosure.

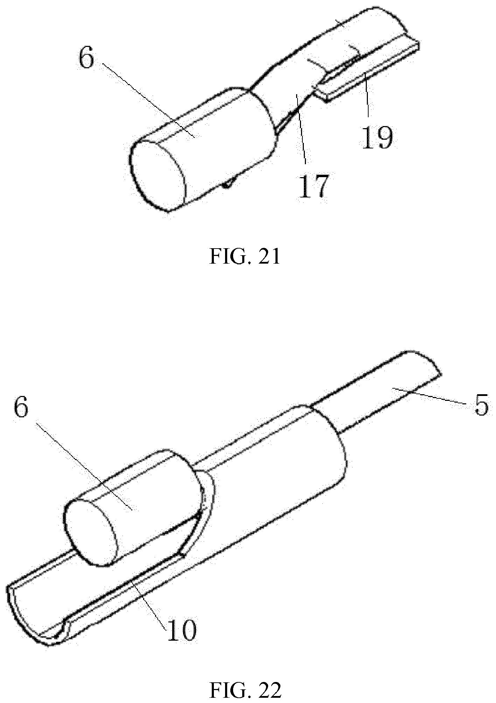

FIG. 21 is a schematic diagram of the inside of a housing of an endoscope with an enlargeable distal end according to Embodiment 17 of the present disclosure.
FIG. 22 is a schematic diagram of a working state of an endoscope with an enlargeable distal end according to Embodiment 17 of the present disclosure.

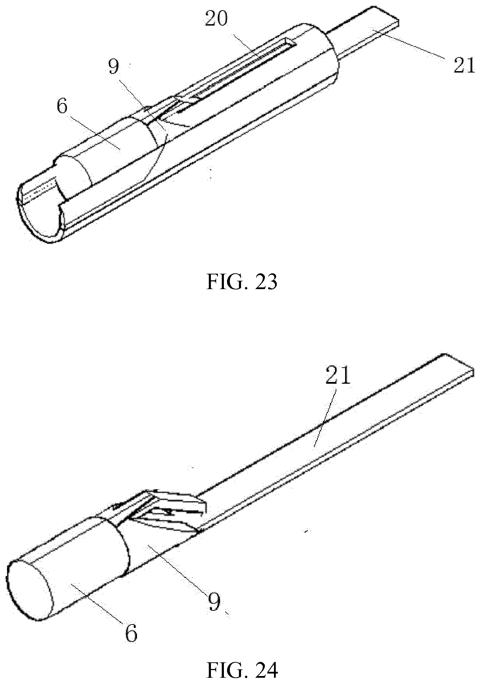

FIG. 23 is a structural diagram of an endoscope with an enlargeable distal end according to Embodiment 20 of the present disclosure.
FIG. 24 is a structural diagram of the inside of a housing according to Embodiment 20 of the present disclosure.

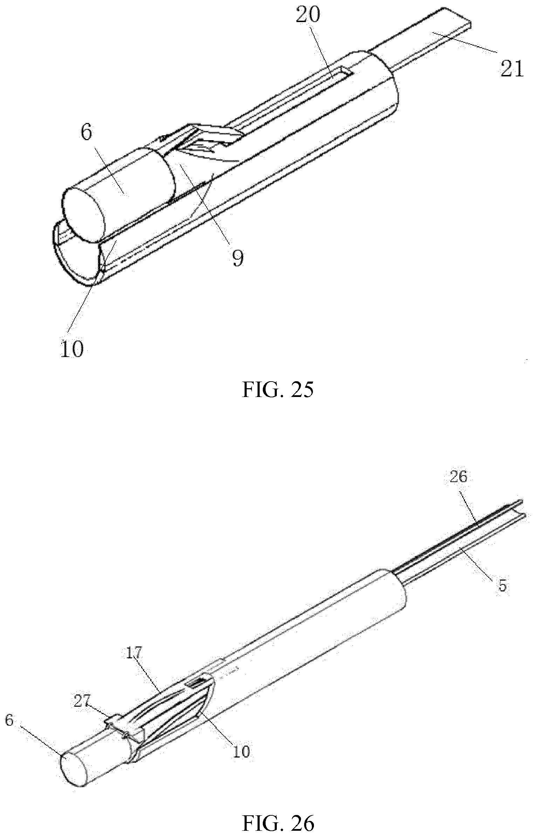

FIG. 25 is a schematic diagram of a working state of an endoscope with an enlargeable distal end according to Embodiment 20 of the present disclosure.
FIG. 26 is a structural diagram of an endoscope with an enlargeable distal end according to Embodiment 22 of the present disclosure.

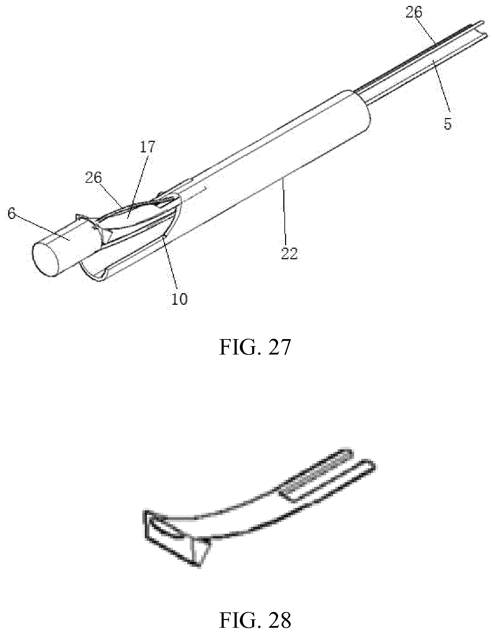

FIG. 27 is a schematic diagram of a working state of an endoscope with an enlargeable distal end according to Embodiment 22 of the present disclosure.
FIG. 28 is a structural diagram of an elastic plate according to Embodiment 22 of the present disclosure.

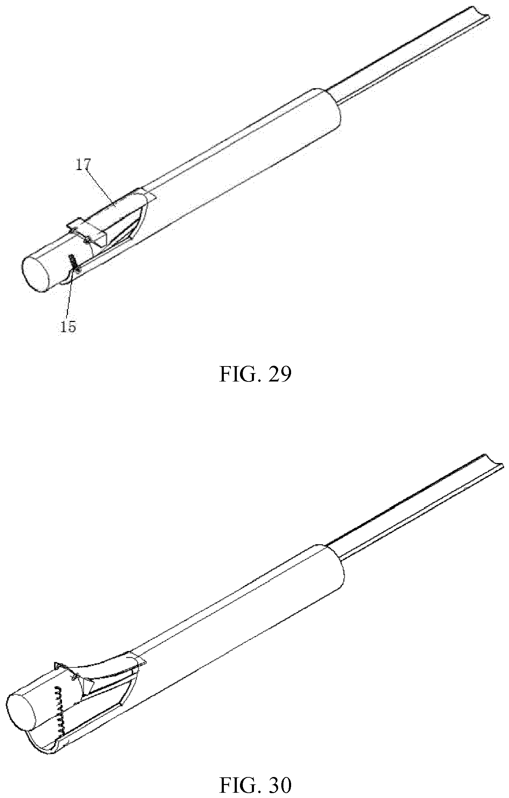

FIG. 29 is a structural diagram of an endoscope with an enlargeable distal end according to Embodiment 23 of the present disclosure.
FIG. 30 is a schematic diagram of a working state of an endoscope with an enlargeable distal end according to Embodiment 23 of the present disclosure.

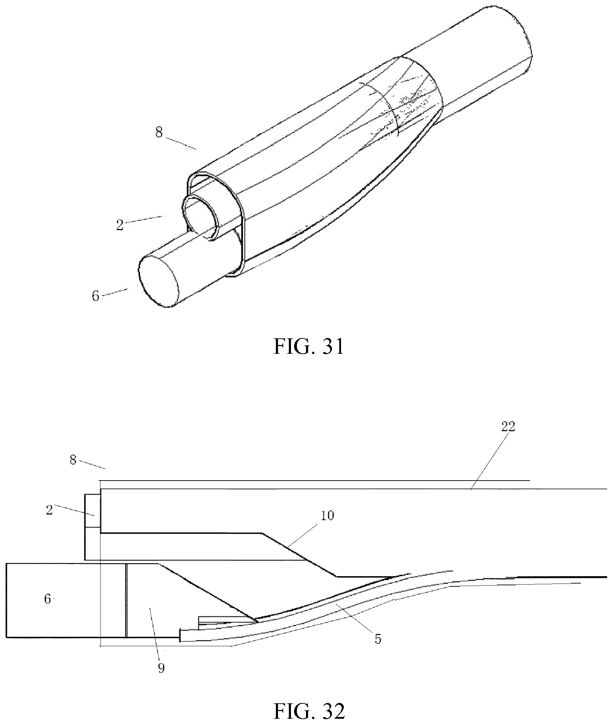

FIG. 31 is a schematic diagram of a working state of an endoscope with an enlargeable distal end according to Embodiment 24 of the present disclosure.
FIG. 32 is a section view of Embodiment 24.
FIG. 33 is a section view of Embodiment 25.

In the drawings, 1 denotes an endoscope main body, 2 denotes a working cavity, 3 denotes a locking button, 4 denotes a housing, 5 denotes a wire band, 6 denotes a camera, 7 denotes an inserting portion, 8 denotes an elastic sleeve, 9 denotes a wedge block, 10 denotes a notch in the housing, 11 denotes a semi tube, 12 denotes a circular tube, 13 denotes an oblique tube, 14 denotes a pull rope, 15 denotes a connecting spring, 16 denotes an expansion support rod, 17 denotes an elastic plate, 18 denotes a steel plate, 19 denotes a partition plate, 20 denotes a sliding groove, 21 denotes an wire band containing a push rod, 22 denotes a housing, 23 denotes a push rod, 24 denotes a rotation ring, 25 denotes an expansion member, 26 denotes a pull rope, and 27 denotes a limit stand.

### DETAILED DESCRIPTION

To better understand the present disclosure, the content of the present disclosure will be further described below in conjunction with embodiments, but is not to be construed as limiting the present disclosure.

### Embodiment 1

An endoscope with an enlargeable distal end includes an inserting portion and an endoscope main body. The inserting portion includes a housing, a working cavity, a camera and an wire band. The working cavity, the camera and the wire band are all disposed in the housing. One end of the wire band is connected to the camera, and the other end of the wire band passes through the housing and is connected to the endoscope main body. The camera is disposed on a distal end in the housing. The distal end of the housing is provided with a notch. The working cavity or the camera can extend out from or retract back into the notch. When the working cavity or the camera extends out from the notch, the working cavity and the camera are in a side-by-side state.

The wire band has a flattened shape and includes wires disposed in parallel and a wrapper wrapping all the wires together.

### Embodiment 2

The Embodiment 2 is almost the same as the Embodiment 1, but the difference is that the endoscope with an enlargeable distal end further includes a wedge block. One end of the wedge block is a plane, and the other end thereof is a bevel. The plane end of the wedge block is connected to the camera, and the bevel end of the wedge block is connected to the working cavity. The working cavity is a tube independent of the housing. The notch in the housing is disposed on one side of the bevel of the wedge block (the notch in the housing is opposite to the bevel of the wedge block). An angle between the bevel of the wedge block and the axis of the wedge block is 30°. When the acting end of the endoscope extends into a target position, the working cavity is pushed, slides out along the bevel of the wedge block, extends out from the notch of the housing, and finally is in a position parallel to the camera. In this case, the medical device can extend into the working cavity to perform various operations. After the operation, the working cavity is pulled back to the starting position and pulled out from the body. The movement of the working cavity relative to the housing may be implemented by setting a notch in the operating end of the housing and pushing the working cavity directly with hands, or may be implemented by adding a working member, one end of which is fixed to the working cavity and the other end of which extends out from the housing, and applying force to the other end to push the working cavity.

### Embodiment 3

The Embodiment 3 is almost the same as the Embodiment 2, but the difference is that the endoscope with an enlargeable distal end further includes an elastic sleeve. The elastic sleeve is disposed at an end portion of one end, provided with the camera, of the housing, and wraps the camera and the notch of the housing. In this way, the effect of protection can be further enhanced.

### Embodiment 4

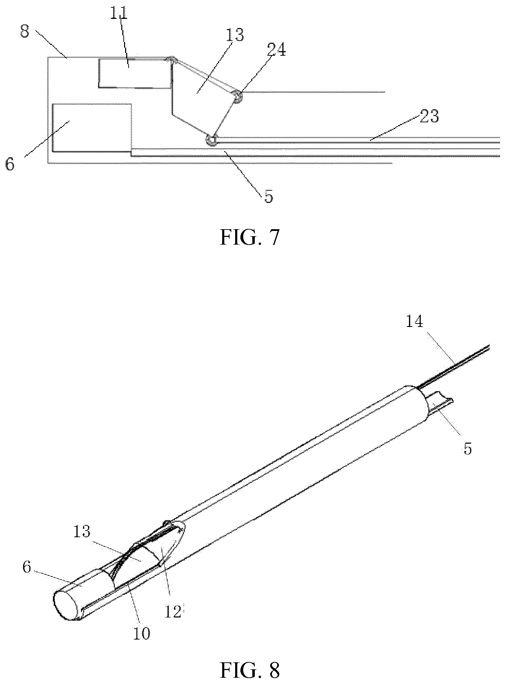

The Embodiment 4 is almost the same as the Embodiment 1, but the difference is that the endoscope with an enlargeable distal end further includes a push rod, an oblique tube and a semi tube. The push rod is disposed in the housing. One end of the oblique tube is a circular surface, and the other end of the oblique tube is a slanting surface. The slanting surface of the oblique tube faces away from the notch of the housing. One side of an end of the circular surface of the oblique tube is rotatably connected to the notch, and the other side of the end of the circular surface of the oblique tube is connected to one end of the push rod. The highest point of one end of the slanting surface of the oblique tube is rotatably connected to one end of the semi tube. One end, which is not connected to the circular tube, of the push rod passes through and extends out from the housing. The cross section of the semi tube is C-shaped. The oblique tube is a circular tube with a surface cut obliquely. The angle between the slanting surface and the axis is 30-60°, and is preferably 45°. As shown in FIG. 7, the slanting surface of the oblique tube can be used as a limit stand when it is in contact with the semi tube.

### Embodiment 5

The Embodiment 5 is almost the same as the Embodiment 4, but the difference is that the endoscope with an enlargeable distal end further includes an elastic sleeve. The elastic sleeve is disposed at the end portion of one end, provided with the camera, of the housing, and wraps the camera. When the acting end of the endoscope extends into the target position, the push rod is pushed to drive the wedge block to slide, and the camera slides away from the notch, so the parts among the semi tube, the oblique tube, the wire band and the housing form the working cavity. In this case, the medical device can extend into the working cavity and perform various operations. After the operation, by pulling the push rod and/or under the compression of the elastic sleeve, the camera restores to the starting position and is pulled out from the body.

### Embodiment 6

The Embodiment 6 is almost the same as the Embodiment 1, but the difference is that the endoscope with an enlargeable distal end further includes a push rod, a connecting spring, a circular tube and a semi tube. The push rod is disposed in the housing. One end of the circular tube is connected to the push rod, and the other end of the circular tube is connected to two end point of one end of the semi tube. One end of the semi tube is attached to the periphery of the camera. One end, which is not connected with the circular tube, of the push rod passes through and extends out from the housing. One end of the connecting spring is connected to the camera, and the other end of the connecting spring is connected to the semi tube. The connecting spring is in a free state. When the acting end of the endoscope extends into the target position, the push rod is pushed, and the circular tube drives the semi tube to move towards the camera and finally pushes the semi tube away from the camera, so the part among the semi tube, the circular tube, the wire band and the housing forms the working cavity. In this case, the medical device can extend into the working cavity to perform various operations. After the operation, by pulling the pull rod and/or with the spring force restored, the circular tube and the semi tube restore to the starting position and then are pulled out from the body.

### Embodiment 7

The Embodiment 7 is almost the same as the Embodiment 1, but the difference is that the endoscope with an enlargeable distal end further includes a pull rope, a circular tube and an oblique tube. One end of the oblique tube is a circular surface and the other end of the oblique tube is a slanting surface. The pull rope is disposed in the housing. One side of an end of the circular tube is rotatably connected to an inner wall of the housing, and the other side of the end of the circular tube is connected to the pull rope. The other end of the circular tube is connected to the circular surface end of the oblique tube. The slanting surface end of the oblique tube is attached to the inner wall of the housing. The camera, the oblique tube, the circular tube and the pull rope are sequentially connected from outside to inside. A rotary connecting point between the circular tube and the housing is on the same side of the notch or on an opposite side of the notch. The circular tube and the oblique rube are disposed in the notch of the housing. When the acting end of the endoscope extends into the target position, the pull rope is pulled, and the circular tube drives the oblique tube to rotate, so the parts among the semi tube, the oblique tube, the wire band and the housing form the working cavity. In this case, the medical device can extend into the working cavity and perform various operations. After the operation, the pull rope is released, and the circular tube and the oblique tube restore to the starting position and then are pulled out from the body. The one side and the other side of one end of the circular tube are two points opposite each other at an end portion of the one end of the circular tube.

### Embodiment 8

The Embodiment 8 is almost the same as the Embodiment 7, but the difference is that the endoscope with an enlargeable distal end further includes an elastic sleeve. The elastic sleeve is disposed at an end portion, provided with the camera, of an end of the housing and wraps the camera, the circular tube and the oblique tube.

### Embodiment 9

The Embodiment 9 is almost the same as the Embodiment 1, but the difference is that the endoscope with an enlargeable distal end further includes an expansion support rod and an elastic sleeve. One end, provided with the camera, of the housing is an elastically bendable end. The elastic sleeve is sleeved on the periphery of the elastically bendable end. One end of the expansion support rod is rotatably connected to the camera, and the other end of the expansion support rod is rotatably connected to the housing. A range of rotation of the expansion support rod relative to the axis of the housing is 30-150°. When the acting end of the endoscope extends into the target position, the wire band is pulled, which makes the camera extend out from the notch of the housing, so the parts between the wire band and the housing form the working cavity. The wire band is continuously pulled, and the limiting function of the expansion support rod makes a periphery of the elastically bendable end a bending structure, so as to achieve the function of a snake-shaped tube. In this case, the medical device can extend into the working cavity and perform various operations. After the operation, the pull rope is released, the elastically bendable end can be restored to the original straight state, and the expansion member restores to the starting position under the extrusion of the elastic sleeve and is pulled out from the body. One end of the wire strap is connected to the camera, the other end is connected to the endoscope body through the housing, the part of the wire strap between the camera and the endoscope body is extended out of the housing, the part of the wire strap is located in the operating portion, easy to pull and on the like operation. One end of the wire band is connected to the camera, the other end of the wire band passes through the housing and is connected to the endoscope main body, a part of the wire band between the camera and the endoscope main body extends out from the housing, and for ease of operation, the extended part of the wire band is typically disposed at the operating portion.

### Embodiment 10

The Embodiment 10 is almost the same as the Embodiment 9, but the difference is that the endoscope with an enlargeable distal end further includes a connecting spring, and is not provided with the elastic sleeve. One end of the connecting spring is connected to the camera, and the other end of the connecting spring is connected to the housing. A compression spring is in the free state. When the connecting spring extends out from the notch of the housing, the connecting spring is in a stretched state. After the operation, the force on the push rod is removed, and the camera restores to the initial position under the resisting force of the connecting spring. Of course, for better protection and use effects, the connecting spring and the elastic sleeve may be simultaneously disposed.

### Embodiment 11

The Embodiment 11 is almost the same as the Embodiment 9, but the difference is that the endoscope with an enlargeable distal end further includes a wedge block. One end of the wedge block is a plane, and the other end of the wedge block is a bevel. The plane end of the wedge block is connected to the camera, and the bevel end of the wedge block is connected to the working cavity. One end of the expansion support rod is rotatably connected to the camera or the wedge block, and the other end of the expansion support rod is rotatably connected to the housing. In this way, the stability can be improved.

### Embodiment 12

The Embodiment 12 is almost the same as the Embodiment 11, but the difference is that the endoscope with an enlargeable distal end further includes an expansion member. The expansion member is disposed on the periphery of the camera and the wedge block. One end of the expansion support rob is rotatably connected to both the camera and the expansion member, and the other end of the expansion support rob is rotatably connected to the housing. In this way, better protection can be achieved.

### Embodiment 13

The Embodiment 13 is almost the same as the Embodiment 1, but the difference is that the endoscope with an enlargeable distal end further includes an expansion support rod, an expansion member and a pull rope. The expansion part is disposed on the periphery of the camera. One end of the expansion support rod is rotatably connected to the expansion member, and the other end of the expansion support rod is rotatably connected to the camera. One end of the pull rope is connected to the expansion member, and the other end of the pull rope passes through and extends out from the housing. When the acting end of the endoscope extends into the target position, the pull rope is pulled, and the expansion meme and the expansion support rod drive the camera to extend out from the notch of the housing, so the parts between the wire band and the housing form the working cavity. In this case, the medical device can extend into the working cavity and perform various operations. The wire band is continuously pulled, and the limiting function of the expansion support rod makes a periphery of the elastically bendable end a bending structure, so as to achieve the function of a snake-shaped tube. In this case, the medical device can extend into the working cavity and perform various operations. After the operation, with the extrusion of the elastic sleeve, the expansion support rod restores to the starting position and then is pulled out the body. One end of the wire band is connected to the camera, and the other end of the wire band passes through the housing and is connected to the endoscope main body. The extended part of the wire band is disposed at the operating portion for ease of operation such as pulling and the like.

### Embodiment 14

The Embodiment 14 is almost the same as the Embodiment 13, but the difference is that the endoscope with an enlargeable distal end further includes a wedge block. One end of the wedge block is a plane, and the other end of the wedge block is a bevel. The plane end of the wedge block is connected to the camera, and the bevel end of the wedge block is connected to the working cavity. One end of the expansion support rod is rotatably connected to the camera or the wedge block, and the other end of the expansion support rod is rotatably connected to the expansion member. In this way, the stability can be improved.

### Embodiment 15

The Embodiment 15 is almost the same as the Embodiment 13, but the difference is that the endoscope with an enlargeable distal end further includes a connecting spring. One end of the connecting spring is connected to the camera, and the other end of the connecting spring is connected to the housing. A compression spring is in the free state. When the connecting spring extends out from the notch of the housing, the connecting spring is in a stretched state. After the operation, the force on the push rod is removed, and the camera restores to the initial position under the resisting force of the connecting spring.

### Embodiment 16

The Embodiment 16 is almost the same as the Embodiment 1, but the difference is that the endoscope with an enlargeable distal end further includes a steel plate and an elastic plate. The steel plate is strip-shaped. The steel plate is slidably connected to the inner wall of the housing. The wire band is disposed between the steel plate and the housing. The elastic plate is shaped. One end of the elastic plate is connected with an end portion of a distal end of the wire band or is connected to the inner wall of the housing that abuts on the distal end of the wire band, and the other end of the wire band is connected to the camera. When the acting end of the endoscope extends into the target position, the steel plate is pushed and acts on the elastic plate, so that the camera deviates from the original position and extends out from the notch, and then the parts among the camera, the steel plate and the housing form the working cavity. In this case, the medical device can extend into the working cavity and perform various operations, the steel plate is pulled and leaves the elastic plate, and then the camera restores to the starting position and is pulled out from the body.

### Embodiment 17

The Embodiment 17 is almost the same as the Embodiment 1, but the difference is that the endoscope with an enlargeable distal end further includes multiple-cavity housing and an elastic plate. The multiple- cavity housing contains at least two cavities. The multiple cavities aggregate at the notch of the housing and form a larger cavity. The elastic plate and the wire band pass in one of the multiple cavities. The end portion of the cavity partition plate is disposed at the notch of the housing. One end of the elastic plate is S-shaped, and the other end of the elastic plate is strip-shaped. The strip-shaped end of the elastic plate is wrapped in the wire band or is in parallel to the wire band. An end portion of the S-shaped end of the elastic plate is connected to the camera. An end portion of the strip-shaped end of the elastic plate is connected to a pushing apparatus on a handle. The distal end of the wire band is connected to the camera. When the endoscope is not working, the camera is retracted in the larger chamber and blocks the rear section of the working cavity. When the acting end of the endoscope extends into the target position, the elastic plate is pulled, and under the blocking of the partition plate and the housing, the elastic S-shaped end becomes straight, so that the camera deviates from the original position and extends out from the notch, leaving the rear section of working cavity. When the endoscope needs to be removed, the elastic plate is pushed, the S-shaped end of the elastic plate is pushed out from the chamber and restores to the S shape, so that the camera is retracted into the larger cavity, restores to the starting position and then is pulled out from the body.

### Embodiment 18

The Embodiment 18 is almost the same as the Embodiment 16, but the difference is that the endoscope with an enlargeable distal end further includes a connecting spring. One end of the connecting spring is connected to the camera, and the other end of the connecting spring is connected to the housing. The connecting spring is in the free state. The connecting spring performs limiting and restoring functions.

### Embodiment 19

The Embodiment 19 is almost the same as the Embodiment 16, but the difference is that the endoscope with an enlargeable distal end further includes an elastic sleeve. The elastic sleeve is disposed at the end portion of one end, provided with the camera, of the housing, and wraps the camera and the elastic plate.

### Embodiment 20

The Embodiment 20 is almost the same as the Embodiment 1, but the difference is that the endoscope with an enlargeable distal end further includes a wedge block and a push rod. One end of the wedge block is a plane, and the other end of the wedge block is a bevel. The plane end of the wedge block is connected to the camera, and the bevel end of the wedge block is slidably connected to the housing. The wedge block and the camera are clamped in the notch of the housing. One end of the push rod is connected to the bevel end of the wedge block and pulls the wedge block to drive the camera to slide relative to the housing, and the other end of the push rod passes through and extends out of the housing.

### Embodiment 21

The Embodiment 21 is almost the same as the Embodiment 20, but the difference is that the endoscope with an enlargeable distal end further includes an elastic sleeve. The elastic sleeve is disposed at the end portion of one end, provided with the camera, of the housing, and wraps the camera. When the acting end of the endoscope extends into the target position, the push rod is pushed to drive the wedge block to slide, and the camera slides away from the notch, so the part between the wire band and the housing forms the working cavity. In this case, the medical device can extend into the working cavity and perform various operations. After the operation, by pulling the push rod and/or under the compression of the elastic sleeve, the camera restores to the starting position and is pulled out from the body.

### Embodiment 22

The Embodiment 22 is almost the same as the Embodiment 1, but the difference is that the endoscope with an enlargeable distal end further includes an elastic plate, a limit stand, a pull rope and an elastic sleeve. The elastic sleeve is strip-shaped. One end of the elastic plate is connected to the inner wall of the housing, and the other end of the elastic plate is connected to the camera and the pull rope. The limit stand is disposed at the joint between the elastic plate and the camera. The pull rope passes through the housing and is connected to a control handle. The elastic sleeve is sleeved at the end portion of one end, provided with the camera, of the housing and wraps the camera. When the acting end of the endoscope extends into the target position, the pull rope is pulled and acts on the elastic plate to make it bend, so that the camera deviates from the original position and extends out from the notch. The limit stand supports the camera. The oblique angle of the limit stand is 15-75°, and is typically 45°. The parts between the wire band and the housing form the working cavity. In this case, the medical device can extend into the working cavity and perform various operations. The pull rope is released, and the camera restores to the starting position under the action of the elastic sleeve and is pulled out from the body.

### Embodiment 23

The Embodiment 23 is almost the same as the Embodiment 22, but the difference is that the endoscope with an enlargeable distal end further includes a connecting spring. The pull rope is wrapped in the wire band. The elastic sleeve is not disposed. The spring replaces the elastic sleeve to make the camera retract. One end of the connecting spring is connected to the camera, and the other end of the connecting spring is connected to the housing. The compression spring is in the free state. When the pull rope is pulled, the wire band is stretched, so that the camera extends out from the notch of the housing. In this case, the connecting spring is in the stretched state. After the operation, the force on the wire band containing the pull rope is removed, and the camera restores to the initial position under the restoring force of the connecting spring. Of course, for better protection and use effects, the connecting spring and the elastic sleeve can simultaneously be disposed.

### Embodiment 24

The Embodiment 24 is almost the same as the Embodiment 1, but the difference is that the endoscope with an enlargeable distal end further includes a wedge block. One end of the wedge block is a plane, and the other end thereof is a bevel. The plane end of the wedge block is connected to the camera, and the bevel end of the wedge block is connected to the working cavity. The working cavity is a tube independent of the housing. The notch in the housing is disposed on one side of the bevel of the wedge block (the notch on the housing is opposite to the bevel of the wedge block). An angle between the bevel of the wedge block and the axis of the wedge block is 15-75° and is typically 45°.When the acting end of the endoscope extends into the target position, the working cavity is pushed forward, the working cavity moves forwards along the bevel of the wedge block, pushes the wedge block and the camera out from the notch of the housing, and is in a position parallel to the camera. In this case, the medical device can extend into the working cavity and perform various operations, and after the operation, the working cavity is pulled back to the starting position and pulled out of the body. The movement of the working cavity relative to the housing may be implemented by setting a notch on the operating end of the housing and pushing the working cavity directly with hands, or may be implemented by adding a working member, one end of which is fixed to the working cavity and the other end of which extends out from the housing, and applying force to the other end to push the working cavity.

### Embodiment 25

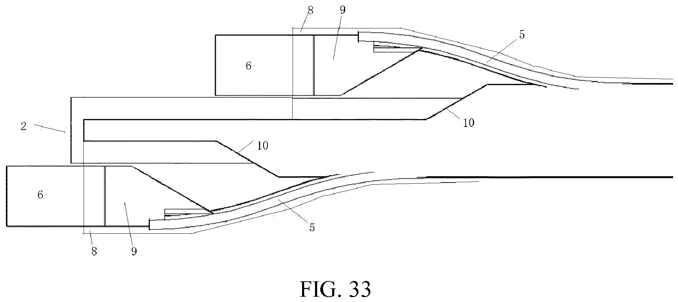

The Embodiment 25 is almost the same as the Embodiment 24, but the difference is that the same endoscope is provided with at least two cameras and wire bands and wedge blocks connected with the at least two cameras. The two cameras are disposed one in front of the other. As shown in FIG. 33, two symmetrical notches are on the housing on the distal end of the endoscope. The elastic sleeves on both sides are in a half-tube structure, and are respectively connected to two different notches. When the acting end of the endoscope extends into the target position, the working cavity is pushed forward along the bevel of the wedge block, pushes the two cameras out from the notches of the housing, and finally is in parallel position with the camera. The two cameras respectively acquire and transmit images to the computer to synthesize 3D images. The two cameras extend out from different notches to work independently without mutual interference.

The two cameras may also be disposed asymmetrically in the endoscope, or may extend out from the same notch.

In one embodiment, the two cameras are respectively a primary camera and a secondary camera, which respectively execute different functions. The primary camera uses a high-definition camera to capture the color image straight in the front, and the secondary camera is behind the primary camera and uses a high-definition camera to capture the color image in the lateral direction. In another embodiment, the primary camera is in front of the secondary camera, the primary camera uses a high-definition camera to capture the color image straight in the front, and the secondary camera uses a special camera to capture images with different spectral bands to display a special lesion. In another embodiment, the main camera is in the front and uses a high-definition camera to capture the color image straight in the front. A circuit in which the secondary camera should be located is no longer provided with a camera, but is connected to other circuitry devices to perform specific functions.

In one embodiment, the front wedge block is provided with a B-mode ultrasound detector, and the rear wedge block is provided with a camera. The B-mode ultrasound detector and the camera respectively perform different functions, which can make the endoscope not only have an optical vision but also detect a position of a tumor in the submucosa.

The endoscope in each of the above embodiments may be manufactured as a disposable device or a reusable device depending on requirements, and the parts not specifically described above are all referred to the related art.

The endoscope with an enlargeable distal end in each of the above embodiments can achieve the enlarging and retracting of the distal end of the endoscope. The distal end of the endoscope is enlarged after passing through a narrow human body tract so that the working cavity or the camera extends out and the working cavity or the camera is in a parallel position. The inner diameter of the working cavity is significantly increased. Therefore, more devices can pass through to take out larger foreign matters, the washing is performed more easily, the maintenance become simple, and the consumption is less; the production cost is low because it can be used once and can also be used multiple times; and structure design is reasonable, and the endoscope is easy to use.

## Claims

1. An endoscope with an enlargeable distal end, comprising an inserting portion and an endoscope main body, wherein the inserting portion comprises a housing, a working cavity, a camera, and an wire band, wherein the working cavity, the camera, and the wire band are all disposed in the housing; one end of the wire band is connected to the camera, and another end of the wire band passes through the housing to be connected to the endoscope main body; the camera is disposed at the distal end in the housing; the distal end of the housing is provided with a notch; the working cavity or the camera is extendable outward from or retractable back into the notch, and when the working cavity or the camera extends out from the notch, the working cavity and the camera are situated side by side.

2. The endoscope of claim 1, wherein when the working cavity or the camera extends out from the notch, an axis of the working cavity and an axis of the camera form an angle that lies in the range of 0 to 30°.

3. The endoscope of claim 1, wherein when the working cavity or the camera extends out from the notch, an axis of the working cavity and the camera run parallel to each other.

4. The endoscope of claim 1, wherein the distal end of the housing is provided with at least two notches, and in a front or rear side of the camera there is arranged at least one surgical component that is extendable outward from or retractable back into the notch.

5. The endoscope of claim 1, wherein the distal end of the housing is provided with at least two notches, and in the same endoscope main body there are arranged at least two cameras that are disposed one in front of the other and that are extendable outward from or retractable back into the notches.

6. The endoscope of claim 1, wherein the distal end of the housing is made of a hard material.

7. The endoscope of claim 1, wherein the distal end of the housing is made of a metal or ceramic.

8. The endoscope of claim 1, wherein a maximum width of the notch measured along a direction perpendicular to a longitudinal axis of the endoscope main body is larger than an outer diameter of the camera.

9. The endoscope of claim 1, wherein a maximum width of the notch measured along a direction perpendicular to a longitudinal axis of the endoscope main body is larger than an outer diameter of the working cavity.

10. The endoscope of claim 1, wherein the wire band has a flattened shape, and comprises wires parallelly arranged and a wrapper that wraps all the wires together; or the wire band is an integrated structure in which the wires are arranged in parallel and bonded to each other.

11. The endoscope of claim 1, further comprising an elastic sleeve that is disposed at the end of the housing where the camera is arranged and that wraps the camera therein.

12. The endoscope of any one of claims 1 to 11, further comprising a wedge block, wherein one end of the wedge block is a plane, and another end of the wedge block is a bevel, the plane end of the wedge block is connected to the camera, the bevel end of the wedge block is connected to the working cavity, wherein the working cavity is a tube independent of the housing, and the bevel of the wedge block faces towards or away from the notch in the housing.

13. The endoscope of any one of claims 1 to 11, further comprising a push rod, an oblique tube and a semi tube, wherein the semi tube has a C-shaped cross section, the push rod is disposed in the housing, one end of the oblique tube is a circular surface, another end of the oblique tube is a slanting surface, which faces away from the notch of the housing; one side of the circular surface end of the oblique tube is rotatably connected to the notch, another side of the circular surface end of the oblique tube is connected to one end of the push rod, a highest point of the slanting surface end of the oblique tube is rotatably connected to one end of the semi tube, and the end of the push rod that is not connected to the circular tube passes through and extends out of the housing.

14. The endoscope of claim 13, further comprising a connecting spring, wherein one end of the connecting spring is connected to the camera or to the housing, another end of the connecting spring is connected to the semi tube, and the connecting spring is in a free state.

15. The endoscope of any one of claims 1 to 11, further comprising a pull rope, a circular tube, and an oblique tube, wherein one end of the oblique tube is a circular surface, another end of the oblique tube is a slanting surface, the pull rope is disposed in the housing; one side of an end of the circular tube is rotatably connected to an inner wall of the housing, another side of the end of the circular tube is connected to the pull rope; another end of the circular tube is connected to the circular surface end of the oblique tube, the slanting surface end of the oblique tube is attached to the inner wall of the housing, wherein the camera, the oblique tube, the circular tube, and the pull rope are sequentially connected to each other in a direction from the outside to the inside, and a rotary connecting point between the circular tube and the housing is located on a same side as the notch or on an opposite side to the notch.

16. The endoscope of any one of claims 1 to 11, further comprising an expansion support rod and an elastic sleeve, wherein the end of the housing provided with the camera is an elastically bendable end, the elastic sleeve is sleeved around a periphery of the elastically bendable end, one end of the expansion support rod is rotatably connected to the camera, another end of the expansion support rod is rotatably connected to the housing, and a range of rotation of the expansion support rod relative to an axis of the housing lies in the range of 0 to 90°.

17. The endoscope of any one of claims 1 to 11, further comprising an expansion member, an expansion support rod, a pull rope, and an elastic sleeve, wherein the end of the housing provided with the camera is an elastically bendable end, the elastic sleeve is sleeved around a periphery of the elastically bendable end; the pull rope is disposed in the housing, one end of the pull rope is connected to the expansion member, another end of the pull rope passes through to be connected to the endoscope main body, the expansion member is disposed on a periphery of the camera, one end of the expansion support rod is rotatably connected to the expansion member, and another end of the expansion support rod is rotatably connected to the housing or the camera.

18. The endoscope of any one of claims 1 to 11, further comprising an expansion support rod, an expansion member, a wedge block, and a push rod, wherein the expansion member is disposed on a periphery of the camera, one end of the expansion support rod is rotatably connected to the expansion member, another end of the expansion support rod is rotatably connected to the camera; one end of the push rod is connected to the expansion member, and another end of the push rod passes through and extends out of the housing; one end of the wedge block is a plane, another end of the wedge block is a bevel, and the plane end of the wedge block is connected to the camera; and a range of rotation of the expansion support rod relative to an axis of the housing lies in the range of 90 to 180°.

19. The endoscope of any one of claims 1 to 11, further comprising a steel plate and an elastic plate, wherein the steel plate is strip-shaped and is slidably connected to the inner wall of the housing, and the wire band is disposed between the steel plate and the housing; one end of the elastic plate is connected to an end portion of the distal end of the wire band or is connected to the inner wall of the housing that abuts on the distal end of the wire band, and another end of the wire band is connected to the camera.

20. The endoscope of any one of claims 1 to 11, further comprising a dual-cavity housing and an elastic plate, wherein the wire band is disposed in one of the two cavities, one end of the elastic plate is S-shaped, another end of the elastic plate is strip-shaped, the strip-shaped end of the elastic plate is wrapped in the wire band, an end portion of the S-shaped end of the elastic plate extends out from the cavity where the wire band is disposed to be connected to the camera, and the camera is disposed in the notch of the housing.

21. The endoscope of any one of claims 1 to 11, further comprising a wedge block and a push rod, wherein one end of the wedge block is a plane, and another end of the wedge block is a bevel, the plane end of the wedge block is connected to the camera, the bevel end of the wedge block is slidably connected to the housing, the wedge block and the camera are clamped in the notch of the housing; one end of the push rod is connected to the bevel end of the wedge block and is operative to push or pull the wedge block to drive the camera to slide relative to the housing, and another end of the push rod passes through and extends out of the housing.

22. The endoscope of any one of claims 1 to 11, further comprising an elastic plate, a pull rope, and an elastic sleeve, wherein one end of the elastic plate is connected to the inner wall of the housing, another end of the elastic plate is connected to the camera and to the pull rope, a limit stand is disposed at a joint of the elastic plate and the camera, wherein the pull rope passes through the housing to be connected to a control handle, and the elastic sleeve is arranged at an end portion of the end of the housing provided with the camera and wraps the camera.
